# EUROPEAN PATENT SPECIFICATION

(11) **EP 0 467 693 B1**
(45) Date of publication and mention of the grant of the patent: **20.01.1999**
(21) Application number: 91306575.1
(22) Date of filing: 19.07.1991
(51) Int. Cl.: G06F 17/30

(54) **Method of, and system for, processing medical information**
Verfahren und Vorrichtung zum Verarbeiten von medizinischen Informationen
Procédé et système pour traiter des données médicales

(30) Priority: 20.07.1990 JP 192409/90; 13.08.1990 JP 213838/90
(43) Date of publication of application: 22.01.1992
(73) Proprietor: NIPPON CONLUX CO., LTD., Chiyoda-ku, Tokyo (JP); Hayakawa, Takenori, Shimotsuga-gun, Tochigi-ken (JP)
(72) Inventor: Furusu, Goro, c/o Galois Corporation, Setagaya-ku, Tokyo (JP)
(74) Representative: Robinson, John Stuart

(56) References cited:
- EP-A- 372 703
- PATENT ABSTRACTS OF JAPAN vol. 12, no. 436 (P-787)17 November 1988 & JP-A-63 167 269 ( CANON INC. ) 11 July 1988
- 3RD USA-JAPAN COMPUTER CONFERENCE PROCEEDINGS 10 October 1978, SAN FRANCISCO, USA pages 441 - 446 A. WATANABE ET AL : 'Small business computers in medical treatment and management systems'

## Description

The present invention relates to a method of, and system for, processing medical information; such a method and system may be used for recording information in the form of letters and/or images obtained from a patient on an optical memory card by a computer, retrieving such information from the optical memory card as prescribed medical information by a computer and utilising the same for management of the patient data.

Medical information has heretofore been controlled mainly by medical record charts prepared by physicians. The chart is usually filled with information concerning patients' medical history, laboratory reports, prescriptions, therapies, etc. in the form of handwritten letters and diagrams, and occasionally in the form of images such as an X-ray film.

As electronic technology has progressed, imaging of medical information has increased. In addition to radiography, such image information includes information obtainable from electrocardiographs, thermographs, nuclear magnetic resonance systems (NMR), CT scanners, ultrasonographs, endoscopes, etc., and greatly contributes to diagnoses and treatments of patients as well as to medical data control.

These image and letter information are stored as they are in the unit of a patient and, whenever necessary, taken out as unprocessed data. As more and more diseases are uncovered by the progress of medicine and the number of patients increases with ageing of the society, the types and quantities of such information have drastically increased.

With the data processing methods presently available, it is quite difficult to retrieve quickly information for each patient for use in his/her examination, and moreover storing of such volume of medical information requires a large memory. If chronological analysis of the history of a disease and treatment of a patient is required to establish optimal policy of treatment, re-processing of the data becomes unavoidable in accordance with the current method.

A flood of information makes it increasingly difficult to sort out and control data, posing problems in quick decision-making or choice of an adequate treatment. Particularly, image data need a larger storage space as they are bulkier than letter data, and this occasionally leads to misplacement or loss of data. As unprocessed image often lacks the required contrast, clearness or size, it could mislead a physician in diagnosing a patient properly.

Document EP-A-0 372 703 describes an information management system and a recording medium used therein. Said system consists of a host, a buffer memory, a disk drive unit, optical disks (each being allocated to a person), a display unit, a center system and a file (containing medical service informationregistered therein for various persons: X-ray photographs, computerized tomograms, etc.), which serve in combination as a medical advice device placed in a hospital.

Each recording medium has a user's area carrying image information related to the possessor of the disk, said user's area includying a specific area carrying a processing program stored therein, so that the processing program is read out from the recording medium an stored in an image processing unit and the image information read out from the recording medium is processed by the image processing unit in accordance with the processing program.

Document PATENT ABSTRACTS OF JAPAN, vol. 12, no. 436 (p-767), 17 November 1988 & JP-A-63 167 269 (CANON INC.), 11 July 1988, describes recording histories of data into a memory medium for an inspector and a memory medium for a person to be inspected by reading and recording said data between the two medium with each other.

The invention relates to a method and to a system as set out in claims 1 and 3 respectively.

It is thus possible to provide a processing method and system for recording, by a computer, various types of medical information in the form of letters or images obtained from a patient on an optical memory card compactly in a predetermined format and recalling such information from time to time whenever a need arises for reproduction.

Medical information processing may be achieved by the steps of writing-in by a computer on a format designated by the computer on an optical memory card information in the form of letter and/or image obtained from a patient, and reading out thus written-in medical information responsive to a command from the computer to thereby reproduce the information arbitrarily, and the image information may be processed for addition, deduction and enlargement of images, adjustment of contrast, scrolling of designated images, multi-frame display as well as for reproduction of the processed images in the form of film.

Medical information may be processed by the steps of inputting in still images in a first computer image information of a patient which is obtained by operation of medical electronic systems, writing-in the input image information on an optical memory card in accordance with the format designated by the first computer, reading-out the written-in image information responsive to a command from a second computer, processing the information responsive to a command from the second computer for addition/deduction/enlargement of images, adjustment in contrast, scrolling of designated images and multi-frame display, and reproducing and displaying the same as the processed image as well as reproducing the processed image in the form of film.

The medical information processing system may comprise an image processing means for converting the analog image information out of the letter and image medical information into the digital data and reproducing the same on a monitor screen, a host computer for inputting the digital image information and/or letter information and transmitting the information to the image processing means for outputting the same on a monitor screen, a writing-in/reading-out means for optical memory cards for writing-in or reading out the medical information in accordance with a format designated by the host computer, and a means for reproducing the processed image information in the form of film so that the image processing means can read out the designated medical information displayed on the host computer from the optical memory card and conduct required processing on the image information thereof.

A medical information processing system may comprise a first image processing means for inputting the medical image information of a patient obtained by an image information acquisition means such as a medical electronic (ME) system as the still image, a first computer for inputting/outputting the image information obtained by the first image processing means, at least one writing-in/reading-out means for optical memory cards for writing-in/reading-out from an optical memory card the image information input in the first computer in a format designated by the first computer, a second computer for inputting/outputting the image information read out by the write-in/read-out means, a second image processing means for processing the image input in the second computer responsive to the command from the second computer for predetermined processing and outputting the same on a monitor screen, and a means for reproducing the image processed by the second processing means in the form of film.

A medical information processing system may comprise a first image processing means for acquiring image information of a patient obtained by an image information acquisition means such as a medical electronic system as the still image, a first computer for inputting/outputting the image information obtained by the first image processing means, a writing-in means for optical memory cards for writing-in the image information input by the first computer in a format designated by the first computer, a reading-out means for reading-out the written-in image information, a second computer for inputting/outputting the read-out image information, a second image processing means for processing the image input by the second computer responsive to a command therefrom and outputting the same on a monitor screen, and a means for reproducing the image processed by the second means in the form of film.

Table 1 is an example list of information on patients and laboratory tests out of the information generally referred to as medical information. Such information is usually expressed in letters (including numerals and codes).

**Table 1.**

| Items. | Details. |
|---|---|
| Urinalysis | pH, protein, ketone, occult blood, urobilinogen, glucose |
| History taking | Past history, family history, subjective symptoms, history of specific diseases (cerebral apoplexy, stenocardia, cardiac infarction, etc.) |
| Measurement of physique | Height, body weight |
| Measurement of blood pressure Blood sampling | Amylase, total protein, albumin, A/G ratio, total bilirubin, direct bilirirubin, thymol reaction, GOT, GPT, γ-GPT, AL-p, LAP, LDH, urea nitrogen, creatinine, etc. |
| Anemia test | RBC |
| Others | Blood type, past history, family history, therapeutic treatment, cytodiagnosis, occult blood, diagnosis. |

Table 2 shows a typical example of image information which is used for laboratory test analysis or diagnosis.

**Table 2.**

| Items. | Remarks. | Image type. |
|---|---|---|
| Simple photography | | |
| Laminagraphy | | Analog X-ray image |
| | | |
| General radiography | | |
| Angiography (head) | | |
| DF | Digital radiography | |
| X-CT (head) | | |
| X-CT (abdomen) | | |
| US | Ultrasonography | Digital X-ray image |
| | | |
| RI (dynamic) | | |
| RI (static) | | |
| NMR | Nuclear magnetic resonance | |
| | | |
| Endoscope | | |

The medical information processing system may, by means of a computer, select appropriate data from the medical information expressed in letters or images as shown in Tables 1 and 2 for a particular patient, write-in the data on an optical memory card, read-out the data from time to time, and reproduce the same in a processed form.

A set of medical information may be substantially input in the computer, and written in an optical card in accordance with the format designated by the computer by a writing-in/reading-out means.

Necessary data may be taken out from the means for writing-in/reading-out responsive to a command output by the computer, displayed, and printed out from an output section such as a printer forming part of the computer system.

All the image information may be input in the computer in the form of digital images. Therefore, for instance, the information which is obtained in the form of analog image such as analog radiographic images listed in Table 2 may be converted by an A/D converter within the image processing means into digital images before being input in said computer.

The image information which is obtained in the form of digital images such as the digital radiographic images of Table 2 and/or letter information may be input in the computer as it is. The image information written in optical memory cards may be displayed as the images which have been processed for a prescribed processing by the image processing means.

More particularly, they may be processed for addition, deduction, enlargement, contrast adjustment, scrolling of designated images, and/or multi-frame display. These processings greatly facilitate diagnosis and decision-making for treatment of diseases.

In addition to the display, the image information stored in the optical memory cards may be displayed/accumulated in the form of 35 mm films or slides whenever necessary. This allows loaning of the stored films to other hospitals or for use in reports at academic meetings.

The image information of a patient obtained through use of ME systems such as a radiograph, an electrocardiograph, a thermograph, an NMR system, a CT scanner, an ultrasonograph and/or an endoscope may be processed as the still digital images processed by the first image processing means, and then transferred to the first computer to be written in optical memory cards by the writing-in/reading-out means. The medical information thus stored may be read out by the writing-in/reading-out means via the second computer. The image information out of the medical information stored by the writing-in/reading-out means may be processed by the second image processing means via the second computer and reproduced/displayed as the processed images.

The second image processing means may execute various types of processing for the image information out of the medical information stored on the optical memory cards such as addition and deduction of images, enlargement, contrast adjustment, scrolling of designated images, and/or multi-frame display. Reproduction and display of these processed images permit accurate diagnosis and treatment.

The processed image obtained by the second image processing means may be loaned to other hospitals for referral purposes or used in reports at academic meetings if reproduced in the form of 35 mm films and slides.

Adminstration of higher efficiency may be realized by dividing the writing-in/reading-out means for optical memory cards into a writing-in means and a reading-out means, and operations up to writing the data in an optical memory card may be conducted by the section in charge of ME systems and image acquisition while the operation of reading-out the data and thereafter may be conducted by departments in charge of treatments.

If the content of ID card assigned to each patient by the hospital is included in the optical memory card in addition to the image information, more accurate information will be available to further improve efficiency in medical data management.

This present invention will be further described, by way of example, with reference to the accompanying drawings in which:

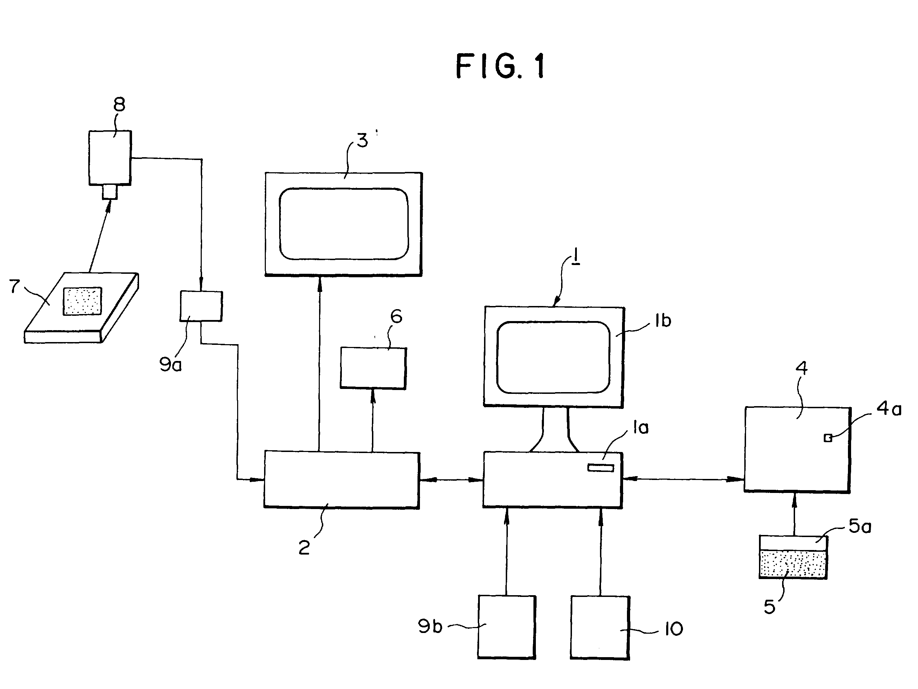

Figure 1 is a schematic diagram of a medical data processing system;

Figure 2 is a schematic diagram of another medical data processing system; and

Figure 3 shows a further schematic diagram of a medical data processing system.

The processing system comprises a host computer 1, an image processing means 2, a monitor 3 comprising a TV monitor, a writing-in/reading-out means 4 for optical memory cards, an optical memory card 5 and a means for reproducing the data in film comprising a video copier.

This embodiment further includes a light transmitting box 7 and a CCD camera 8 which takes pictures of photographic images placed on the box 7. The box 7 and the CCD camera 8 may be replaced by other image transmission means.

The host computer 1 comprises a central processor 1a having functions of arithmetic operation, a display 1b, an input unit comprising a keyboard (not shown) and an output unit comprising a printer, and inputs the medical information such as digital image information 9b and/or letter information 10 directly.

Analog images 9a photographed by the CCD camera 8 are converted from analog to digital by the image processing means 2 and written-in by a means for writing in/reading out on an optical memory card. The host computer 1 includes a function to read the designated data and transfer the same to the image processing means 2.

The image processing means 2 converts the analog image information taken in by the CCD camera 8 from analog to digital. The converted images are transmitted to the host computer 1, processed for various types of image processing designated by the commands from the host computer 1 and reproduced on the monitor 3.

The means 4 writes-in the information on an optical memory card 5 or reads the data therefrom responsive to commands from the host computer 1.

The means 4 is activated and operated by the commands from the host computer 1 and, therefore, has no special manipulating means other than a push button 4a for start/stop.

The optical memory cards 5 are the media for recording the image or letter information. The card is as big as a name card and has a space 5a at a corner of the surface for indicating the names of the hospital and the patient by printing, hand-writing or a label.

The reproducing means 6 is adapted to reproduce the images outputted on the monitor screen 3 in the form of 35 mm films or slides to be used for academic reports or loaned to other hospitals.

The CCD camera 8 takes pictures of the radiograph placed on the light transmission box 7 and transfers thus taken-in images to the image processing means 2 as analog images.

The processing method for medical information using above mentioned system will now be explained below.

The medical information which is to be recorded by the system mainly includes the letter information listed in Table 1 and the analog/digital image information listed in Table 2. For example, in the case of analog data such as a radiograph, a radiograph is placed on the light transmission box 7, photographed by the CCD camera 8, transferred to the image processing means 2 in the form of analog image information 9a, converted from analog to digital by the means 2, and displayed in digital form on the monitor 3 as well as transmitted to the host computer 1.

The image information 9b which is directly obtained in the form of digital radiographic images can be input together with the letter information 10 in the host computer 1 and, whenever necessary, may be taken out alone on the monitor 3 via the image processing means 2. The letter information 10 may be input in the host computer 1 via the keyboard.

The medical information comprising the analog image information 9a converted from analog to digital by the means 2, the image information 9b obtained in the digital form, and the letter information 10 is written in an optical memory card 5 in accordance with a predetermined format by the writing-in/reading-out means 4 via the host computer 1. The information thus recorded on the card 5 is read out by the means 4.

The read-out information is displayed as the designated information on the display 1b responsive to the command from the host computer 1 and may be printed out by a printer, if it is letter information.

The image information 9a and 9b thus read-out are transmitted to the image processing means 2 for image processing of the data output on the monitor 3.

Image processing is performed as follows: They are processed responsive to the command from the host computer 1.
1 Arithmetic operation of images
   By adding and/or deducting data of different radiographs, progress of a disease or lesions may be observed.
2 Zoom-up of the output image on the screen
   Diagnosis is facilitated if the lesion or a site difficult to determine is enlarged.
3 Contrast adjustment
   If the photographed image is too dark or too light, the contrast may be adjusted to reproduce the image optimally.
4 Scrolling of designated frames
   If a specific site is out of the frame, frames may be scrolled to reveal hidden portions.
5 Multi-frame display of images
   Up to four frames may be simultaneously displayed on one screen, for example, to display the progress of a disease or chronological radiographic changes for better chronological control of patient data. The image information which has been processed by the means 2 may be stored in 35 mm films by a means for reproduction 6.

The optical memory card 5 includes a space 5a for indicating names of the hospital, the patient, etc. The format for inputting the data comprises a patient data section for inputting the name, date of birth, sex, date of first visit, etc. of the patient, a directory section for selecting the data on the card, an index section, a data control section and a storage section for the data per se.

Instead of photographing by the CCD camera 8 of the radiograph placed on the light transmission box 7, the radiograph may be directly converted by the image processing means from analog to digital and written in on the optical memory card by the host computer to further rationalize the processing steps.

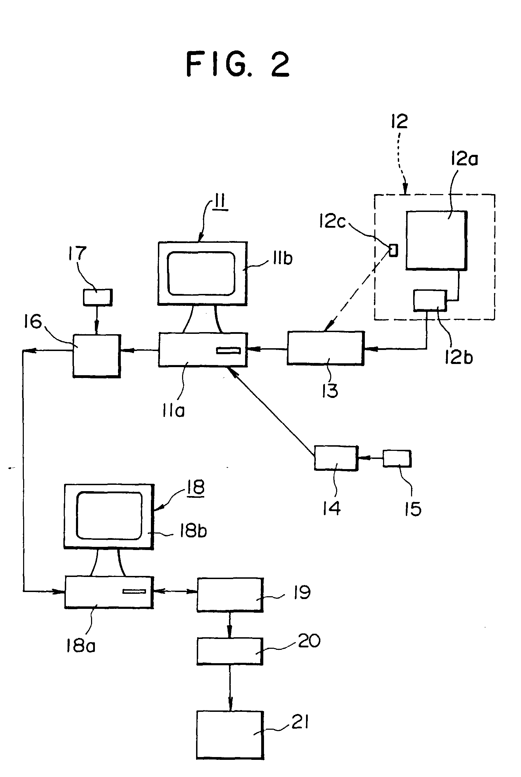

Figure 2 shows an example of a second system for medical information processing.

This embodiment comprises a first computer 11, an image information acquisition means 12 for obtaining the patient data from various types of ME systems 12a, a first image processing means 13 which processes the dynamic data obtained from the means 12 into still images, an ID card reading means 14 which reads entries on the ID card 15 issued to each patient by the hospital, a writing-in/reading-out means 16 which inputs in the first computer 11 the still image data obtained from the image processing means 13 and the letter information obtained from the ID card 15 via the means 14, processes the data for arithmetic operations by the first computer 11, outputs in a predetermined format, writes-in on an optical memory card 17, and reads-out the same, a second computer 18 which inputs thus read-out medical information by the means 16 from the optical memory card, a second image processing means 19 which processes them responsive to the second computer 18 and outputs the same on a monitor 21, and a means 20 for reproducing the images processed by the second means 19 in the form of films.

The first and the second computers 11, 18 respectively comprise central processing units (CPU) 11a, 18a with arithmetic operation functions, displays 11b, 18b input units comprising keyboards (not shown), and output units such as printers.

The medical information acquisition means 12 includes an ME system 12 which has a function to examine and take out the result as dynamic images. The ME system may include a radiograph, an electrocardiograph, a thermograph, an NMR system, a CT scanner, an ultrasonograph, an endoscope, etc. The image information obtained by such systems are input at the first image processing means 13 via a monitor 12b.

The first image processing means 13 can process the dynamic images obtained by the means 12 to be still images by manipulating the switch 12c while viewing the display on the monitor 12b and converting the analog images obtained by the means 12 to digital images while processing the digital images as still digital images without conversion. The image information obtained by this processing are transmitted to the first computer 11.

The ID card reading means 14 reads the contents of an ID card 15 (usually of the size similar to a name card) which is issued to a patient from a hospital. The readout information is input as the letter information at the CPU 11a of the first computer 11. The letter information input at the CPU 11a of the first computer 11 may be reproduced and displayed at the display 11b.

The optical memory card writing-in/reading-out means 16 writes in the medical information comprising the letters and the images output from the first computer 11 on an optical memory card 17. Thus written-in medical information is transmitted to the second computer 18 based on a command from the computer 18. The means 16 is operative when it receives a command from the second computer 18, and therefore has no manipulation means.

The optical memory card 17 is a medium for recording the image information obtained by the means 12 and the letter information recorded on the ID card 15, and usually has the size of a name card.

The second image processing means 19 transmits the image information out of the medical information on the card 17 which has been written-in/read-out by the means 16 to the second computer 18, and processes the image for predetermined processing based on the command from the computer 18. The processed images are reproduced and displayed on a monitor 21 such as TV monitor screen.

The reproducing means 20 can reproduce the images output on the monitor 21 in the form of 35mm films or slides for loan to other hospitals for referral purposes or used in academic reports at meetings. If such film reproduction is not necessary, the images are displayed only on the monitor 21.

An example of the processing method of medical information by using the above steps comprehensively will now be described below.

The medical information which is to be processed by the system shown in Figure 2 as a flow chart is mainly the image information obtained by the image information acquisition means 12. In order to confirm that the image information concerns a/the particular patient without error, it is preferable that the image information be compared with the content of the ID card 15 of the particular patient, and the requisite content of the card 15 is included in the entry of the optical memory card 17, to thereby improve efficiency of the operation.

More particularly, the content of the ID card 15 is read out by the means 14, transmitted to the first computer 11 and displayed on the display 11b. The displayed content is written-in by the means 16 on the card 17 in the format designated by the first computer 11.

The image information of the patient obtained by the means 12 comprising various ME systems 12a is directly input into the computer 11 as the still digital image by the means 13 by manipulating the switch 12c of the means 12 and written in the card 17 via the means 16 in a prescribed format.

The medical information comprising the letter information and the image information recorded on the card 17 is read out by the means 16 from the card 17 and displayed by the command from the second computer 18 on the display 18b.

The image information out of the read-out information is transmitted to the second image information processing means 19 for predetermined processing and output to the monitor 21 via the reproducing means 20.

The means 19 can perform the processing steps (1) to (5) mentioned above responsive to a command from the second computer 18.

The image information processed by the means 12 may be reproduced in 35 mm films, etc. by the reproducing means 21 for loan to other hospitals or use in academic meetings.

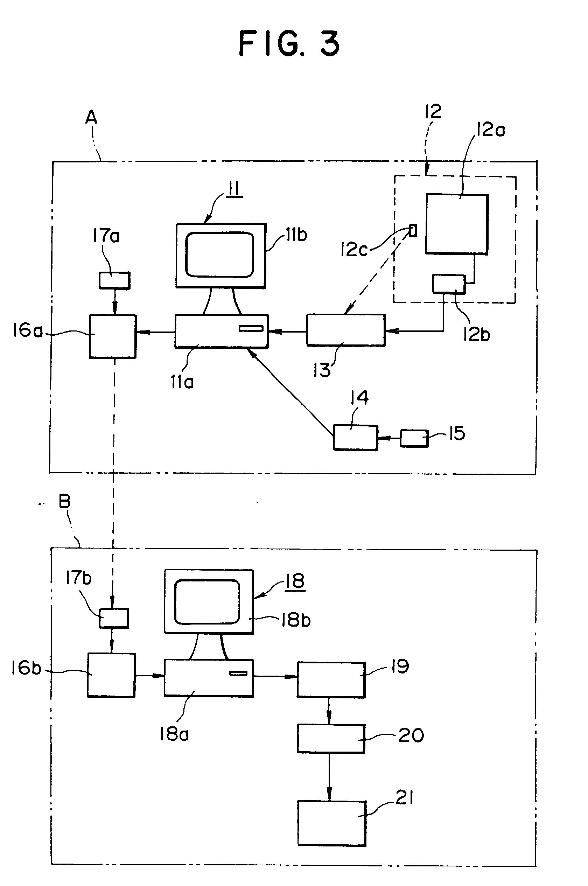

Figure 3 shows a processing system suitable for use with the second processing method. As shown in Figure 3, this embodiment divides the writing-in/reading-out means 16 of Figure 2 into a writing-in means 16a and a reading-out means 16b. The parts up to the step of writing-in on the optical memory card are referred to as the image recording section A and the latter from the reading-out of the card by the means 16b as the image reproducing section B. The other parts of the structure except for the parts A and B are identical to those shown in Figure 2. The same component parts are denoted with the same reference codes and numerals in both figures except for the means 16a, 16b and the optical memory card to avoid duplication in description.

At hospitals, generally speaking, specialists such as radiation technicians are usually in charge of ME systems 12a and for acquiring images while department physicians diagnose patients based on acquired information.

The medical information processing system shown in Figure 3 is so structured as to facilitate medical data control with higher efficiency as the image information acquired by the technicians is written in the card 17a by the means 16a, and the card 17b is read out by a reading-out means 16b in the image reproduction section B positioned at each department of the hospital. Although the number of machines and equipment increases in this structure, merits obtained by the separate arrangement more then offset demerits.

The use of the ID card 15 and the reading-out means 14 for ID cards shown in Figures 2 and 3 is not always required.

The medical information processing method described hereinbefore enables writing-in the medical information in the form of letters and/or images obtained from a patient by a computer in an optical memory card in the format designated by the computer, reproducing responsive to a command from the computer by reading-out the information from the card, and processing of the image information for predetermined image processings, or reproducing in the form of film. A large volume of medical information may be recorded in such an optical memory card compactly thereby to simplify remarkably the storage and administration of medical data.

The medical information recorded on an optical memory card may be quickly and accurately reproduced and displayed by means of a computer. As this system conducts image processing on the image information particularly, such information can be recognized very clearly to eliminate possible errors in diagnosis and to enhance remarkably accuracy in medical judgement.

As the optical memory cards may accumulate a large volume of medical information, chronological control of patients is conveniently facilitated.

The processing system for medical information comprises a computer, an optical memory card, a means for writing-in/ reading-out of information on the optical memory card, an image processing means for processing image information, reproducing and displaying the same in the prescribed form, and a means for reproducing the processed images in the form of films, despite such simple structure of the system, the system can store a large volume of medical information on the card.

This processing system is characterized in that the operation thereof is remarkably simplified by structuring the system in such a manner that medical information is input in an optical memory card by a command from a computer and output/displayed thereby to contribute greatly to processing of ever increasing medical information. As the optical memory card cannot be erased or its data thereon modified without instruction, the information thereon may be reserved without change.

The processing of medical information enables image information of an individual patient obtained by image information acquisition means comprising ME systems for processing as still images by the first image processing means, to be transferred to the first computer, written in an optical memory card by a command from the first computer, read out and reproduced by a command from the second computer, and processed for prescribed image processing by an image processing means for reproducing the information in an optimal form for examination, diagnosis or reproduction in the form of film.

The optical memory cards used herein can store a large volume of medical information compactly as well as remarkably simplify the storage and adminstration of medical data.

The medical information stored in the optical memory cards can be quickly as well as precisely reproduced or displayed by the second computer. Since image information is processed by the method, it can be accurately recognized and can eliminate errors in diagnosis.

If the image information obtained by said image information acquisition means is combined with the information of an individual patient obtained from his/her ID card when such a need arises, errors may be prevented and optimal operation is realized.

As these optical memory cards can accumulate a large volume of medical information data on patients, they are conveniently controlled chronologically.

A processing system for medical information may comprise an acquisition means comprising ME systems, a first image processing means for processing the obtained images as still images, a means for writing-in/reading-out the information in and out of an optical memory card via the first computer, a second computer for inputting/outputting the image information read out of the optical memory card, an image processing means for processing the information into prescribed images by said second computer, and a reproducing means for reproducing and displaying the images processed by the processing means in the form of film.

The construction mentioned above permits adequate recording of a large volume of medical information in the optical memory cards. If the image information acquisition means comprising ME systems can be adapted for processing by computers, image information optimal for diagnosis can be obtained easily and rapidly.

Thus medical information can be stored in an optical memory card based on the instruction from respective computers and reproduced in a predetermined format, to thereby handle medical information quite effectively without help from outside.

As optical memory cards cannot be erased or the contents corrected without authorization, it is possible to store securely and reserve the information input information as it is.

## Claims

1. A method of processing medical information, comprising the steps of (i) inputting image information on medical treatment of a patient obtained by manipulation of medical electronic (ME) systems (12a) as a still image in a first computer (11), (ii) writing in the input information onto an optical memory card (17) in accordance with a format designated by the first computer (11), (iii) reading out the image information responsive to a command from a second computer (18), (iv) processing the images for at least one process of addition, deduction, enlargement, contrast adjustment, scrolling of designated images, and multi-frame display responsive to a command from the second computer (18) for reproducing/displaying, and (v) reproducing the processed images in the form of films.

2. A processing method as claimed in claim 1, characterized in that the optical memory card (17) is written-in with the image information via the first computer (11) as well as letter information on a patient identification card (15).

3. A system for processing medical information, comprising:
a first image processing means (13) for acquiring as a still image information on medical treatment of a patient obtained by an image information acquisition means (12);
a first computer (11) for inputting/outputting the image information obtained by the first image processing means (13);
a writing-in means (16a) for writing the input image information of the first computer (11) in a format designated by the first computer (11) onto an optical memory card (17);
a reading-out means (16b) for reading out the written image information;
a second computer (18) for inputting/outputting the input image information read out by the reading-out means (16b);
a second image processing means (19) for processing the image information input in the second computer (18), for at least one process of addition, deduction, enlargement, contrast adjustment, scrolling of designated images and multi-frame display, responsive to a command from the second computer (18) and outputting the input image information; and
a reproducing means (20) for reproducing the image which has been processed by the second image processing means in the form of a film.

4. A system for processing medical information as claimed in claim 3, characterized in that there is further provided a reading-out means (14) which reads the letter information out of a patient identification card (15) and writes the read letter information onto an optical memory card via the first computer (11).

## Patentansprüche

1. Verfahren zur Verarbeitung von medizinischer Information mit den Schritten; (i) Eingeben von Bildinformation uber medizinische Behandlung eines Patienten, die durch Handhabung von medizinischen elektronischen (ME-)Systemen (12a) gewonnen wird, als stehendes Bild in einen ersten Computer (11), (ii) Schreiben der Eingangsinformation auf eine optische Speicherkarte (17) entsprechend einem Format, das von dem ersten Computer (11) bestimmt wird, (iii) Lesen der Bildinformation als Antwort auf einen Befehl von einem zweiten Computer (18), (iv) Verarbeiten der Bilder für mindestens einen Vorgang der Addition, Subtraktion, Vergrößerung, Kontrösteinstellung, des Rollens von bestimmten Bildern und der Mehrfach-Einzelbildanzeige als Antwort auf einen Befehl von dem zweiten Computer (18) zur Wiedergabe/Anzeige und (v) Wiedergabe der verarbeiteten Bilder in Form von Filmen.

2. Verarbeitungsverfahren nach Anspruch 1, dadurch gekennzeichnet, daß die optische Speicherkarte (17) durch den ersten Computer (11) mit der Bildinformation sowie mit Schriftzeicheninformation auf einer Patientenidentiflkationskarte (15) beschrieben wird.

3. System zur Verarbeitung von medizinischer Information mit:
einer ersten Bildverarbeitungseinrichtung (13) zur Erfassung einer Stehbildinformation über medizinische Behandlung eines Patienten, die von einer Bildinformationserfassungseinrichtung (12) gewonnen wird;
einem ersten Computer (11) zum Eingeben/Ausgeben der Bildinformation, die von der ersten Bildverarbeitungseinrichtung (13) gewonnen wird;
eine Schreibeinrichtung (16a) zum Schreiben der Eingabebild-information des ersten Computers (11) in einem Format, das von dem ersten Computer (11) bestimmt wird, auf eine optische Speicherkarte (17);
einer Leseeinrichtung (16b) zum Lesen der geschriebenen Bild-information;
einem zweiten Computer (18) zum Eingeben/Ausgeben der Eingangsbildinformation, die von der Leseeinrichtung (16b) gelesen wird;
einer zweiten Bildverarbeitungseinrichtung (19) zum Verarbeiten der in den zweiten Computer (18) eingegebenen Bildinformation für mindestens einen Vorgang der Addition, Subtraktion, Vergrößerung, Kontrasteinstellung, des Rollens der bestimmten Bilder und der Mehrfach-Einzelbildanzeige als Antwort auf einen Befehl von dem zweiten Computer (18) und Ausgeben der Eingangsbildinformation; und
einer Wiedergabeeinrichtung (20) zur Wiedergabe des Bildes, das von der zweiten Bildverarbeitungseinrichtung verarbeitet worden ist, in Form eines Films.

4. System zur Verarbeitung von medizinischer Information nach Anspruch 3, dadurch gekennzeichnet, daß ferner eine Leseeinrichtung (14) vorgesehen ist, die die Schriftzeicheninformation von einer Patientenidentifikations-karte (15) liest und durch den ersten Computer (11) gelesene Schrift-zelcheninformation auf eine optische Speicherkarte schreibt.

## Revendications

1. Procédé de traitement d'une information médicale, comprenant les étapes de (i) entrée d'une information d'image d'un traitement médical d'un patient, obtenue en manipulant des systèmes électroniques médicaux (ME) (12a), en tant qu'image fixe dans un premier ordinateur (11), (ii) écriture de l'information d'entrée sur une carte mémoire optique (17) conformément à un format désigné par le premier ordinateur (11), (iii) lecture de l'information d'image en réponse à une commande provenant d'un second ordinateur (18), (iv) traitement des images pour au moins un processus pris parmi une addition, une soustraction, un agrandissement, un réglage de contraste, un défilement d'images désignées et un affichage multi-image en réponse à une commande provenant du second ordinateur (18) pour reproduire/afficher et (v) reproduction des images traitées sous la forme de films.

2. Procédé de traitement selon la revendication 1, caractérisé en ce que la carte mémoire optique (17) reçoit en écriture l'information d'image via le premier ordinateur (11) ainsi qu'une information sous forme de lettres sur une carte d'identification de patient (15).

3. Système de traitement d'une information médicale, comprenant:
des premiers moyens de traitement d'image (13) pour acquérir en tant qu'image fixe une information concernant un traitement médical d'un patient obtenue à l'aide de moyens d'acquisition d'information d'image (12);
un premier ordinateur (11) pour entrer/émettre en sortie l'information d'image obtenue au moyen des premiers moyens de traitement d'image (13);
des moyens d'écriture (16a) pour écrire l'information d'image d'entrée du premier ordinateur (11) selon un format désigné par le premier ordinateur (11) sur une carte mémoire optique (17);
des moyens de lecture (16b) pour lire l'information d'image écrite;
un second ordinateur (18) pour entrer/émettre en sortie l'information d'image d'entrée lue par les moyens de lecture (16b);
des seconds moyens de traitement d'image (19) pour traiter l'information d'image entrée dans le second ordinateur (18) pour au moins un processus pris parmi une addition, une soustraction, un agrandissement, un réglage de contraste, un défilement d'images désignées et un affichage multi-image en réponse à une commande provenant du second ordinateur (18) et pour émettre en sortie l'information d'image d'entrée; et
des moyens de reproduction (20) pour reproduire l'image qui a été traitée par les seconds moyens de traitement d'image sous la forme d'un film.

4. Système de traitement d'une information médicale selon la revendication 3, caractérisé en ce que sont en outre prévus des moyens de lecture (14) qui lisent l'information sous forme de lettres a partir d'une carte d'identification de patient (15) et qui écrivent l'information sous forme de lettres lue sur une carte mémoire optique via le premier ordinateur (11).
